# EUROPÄISCHE PATENTSCHRIFT

(11) **EP 1 440 190 B1**
(45) Veröffentlichungstag und Bekanntmachung des Hinweises auf die Patenterteilung: **17.12.2008**
(21) Anmeldenummer: 02778982.5
(22) Anmeldetag: 30.10.2002
(51) Int. Cl.: D01F 11/00

(54) **VERFAHREN ZUR BEHANDLUNG VON LÖSUNGSMITTELGESPONNENEN CELLULOSISCHEN FASERN**
METHOD FOR TREATING SOLVENT-SPUN CELLULOSE FIBERS
PROCEDE POUR TRAITER DES FIBRES CELLULOSIQUES FILEES AU SOLVANT

(30) Priorität: 02.11.2001 AT 17312001
(43) Veröffentlichungstag der Anmeldung: 28.07.2004
(73) Patentinhaber: LENZING AKTIENGESELLSCHAFT, 4860 Lenzing (AT)
(72) Erfinder: RENFREW, Andrew Hunter Morris, Bury, Lancashire BL8 4DT (GB)
(74) Vertreter: Nemec, Harald
(86) Internationale Anmeldenummer: PCT/AT2002/000306
(87) Internationale Veröffentlichungsnummer: WO 2003/038164

(56) Entgegenhaltungen:
- WO-A-94/09191
- WO-A-95/28516
- FR-A- 2 273 091
- PATENT ABSTRACTS OF JAPAN vol. 1999, no. 09, 30. Juli 1999 (1999-07-30) & JP 11 092435 A (TORAY INDUSTRIES), 6. April 1999 (1999-04-06)

## Beschreibung

Die Erfindung betrifft ein Verfahren zur Behandlung von Cellulosefasern und von Gebilden aus diesen Cellulosefasern, bei welchem Verfahren die Fasern bzw. die Fasergebilde mit einem Textilhilfsmittel in Kontakt gebracht werden, um den Fasern verbesserte Eigenschaften zu verleihen.

Als Alternative zum Viskoseverfahren wurden in den letzten Jahren eine Reihe von Verfahren beschrieben, bei denen Cellulose ohne Bildung eines Derivats in einem organischen Lösungsmittel, einer Kombination eines organischen Lösungsmittels mit einem anorganischen Salz oder in wäßriger Salzlösung gelöst wird. Cellulosefasern, die aus solchen Lösungen hergestellt werden, heißen "lösungsmittelgesponnene" Fasern und erhielten von der BISFA (The International Bureau for the Standardisation of man made Fibres) den Gattungsnamen Lyocell. Als Lyocell wird von der BISFA eine Cellulosefaser definiert, die durch ein Spinnverfahren aus einem organischen Lösungsmittel erhalten wird. Unter "organisches Lösungsmittel" wird von der BISFA ein Gemisch aus einer organischen Chemikalie und Wasser verstanden. "Lösungsmittelspinnen" soll Auflösen und Spinnen ohne Derivatisierung bedeuten.

Bis heute hat sich jedoch nur ein einziges Verfahren zur Herstellung einer lösungsmittelgesponnenen Cellulosefaser bis zur industriellen Realisierung durchgesetzt. Bei diesem Verfahren wird als Lösungsmittel ein tertiäres Aminoxid, insbesondere N-Methylmorpholin-N-oxid (NMMO), verwendet. Ein solches Verfahren ist z.B. in der US-A 4,246,221 beschrieben und liefert Fasern, die sich durch eine hohe Festigkeit, einen hohen Naßmodul und durch eine hohe Schlingenfestigkeit auszeichnen.

Die Brauchbarkeit von Flächengebilden, z.B. Geweben, hergestellt aus den genannten Fasern, wird jedoch durch die ausgeprägte Neigung dieser Fasern, im nassen Zustand zu fibrillieren, stark eingeschränkt. Unter Fibrillierung wird das Aufbrechen der Faser in Längsrichtung bei mechanischer Beanspruchung im nassen Zustand verstanden, wodurch die Faser ein haariges, pelziges Aussehen erhält. Ein aus diesen Fasern hergestelltes und gefärbtes Gewebe verliert im Laufe weniger Wäschen stark an Farbintensität. Dazu kommt noch, daß sich an Scheuer- und Knitterkanten helle Streifen ausbilden. Als Ursache für die Fibrillierung wird angenommen, daß die Faser aus in Faserrichtung angeordneten Fibrillen besteht, zwischen denen nur in geringem Ausmaß eine Quervernetzung vorhanden ist.

Ferner kann es auch bei der Färbung der Fasern in Strangform zur Streifenbildung kommen. In textilen Flächen kann es durch Reibung im Trockenen zur Bildung von Knötchen kommen, welche Eigenschaft als "Pilling" bekannt ist.

Die WO 92/07124 beschreibt ein Verfahren zur Herstellung einer Faser mit verringerter Fibrillierneigung, gemäß dem die frisch gesponnene, also noch nicht trockene Faser mit einem kationischen Polymer behandelt wird. Als derartiges Polymer wird ein Polymer mit Imidazol- und Azetidin-Gruppen genannt. Zusätzlich kann noch eine Behandlung mit einem emulgierbaren Polymer, wie z.B. Polyethylen oder Polyvinylacetat, oder auch eine Vernetzung mit Glyoxal erfolgen.

In einem bei der CELLUCON-Konferenz 1993 in Lund, Schweden, von S. Mortimer gehaltenen Vortrag wurde erwähnt, daß die Fibrillierneigung mit zunehmender Verstreckung ansteigt.

Die EP-A 0 538 977 und die WO 94/09191 sowie die WO 95/28516 beschreiben ein Verfahren der eingangs erwähnten Art, bei welchem lösungsmittelgesponnene Fasern zur Verringerung der Fibrillierneigung mit einem Textilhilfsmittel in Kontakt gebracht werden.

Die WO 94/24343 beschreibt ein Verfahren zur Herstellung von Cellulosefasern mit verringerter Fibrillierneigung, bei welchem Verfahren eine Lösung von Cellulose in einem tertiären Aminoxid zu Fasern versponnen und die frisch gesponnenen Fasern mit einem Textilhilfsmittel, das mindestens zwei reaktive Gruppen trägt, in Kontakt gebracht und mit einem wässerigen Puffer gewaschen werden, wobei als Textilhilfsmittel nicht Glyoxal eingesetzt wird. Gemäß diesem vorbekannten Verfahren werden die frisch gesponnenen Fasern mit dem Textilhilfsmittel am besten in einem alkalischem Milieu in Kontakt gebracht.

Es ist ferner bekannt, daß Fasergebilde aus lösungsmittelgesponnene Fasern mit Methylolverbindungen vernetzt werden können, um waschstabile Gewebe und Gestricke herzustellen. Es hat sich jedoch gezeigt, daß es nicht möglich ist, bei Verwendung dieser Verbindungen die Bildung von Scheuerkanten bei der Färbung zu verhindern. Dazu müßte die Vernetzung vor der Färbung oder zumindest während der Färbung erfolgen. Methylolverbindungen und auch die anderen klassischen Hochveredelungsmittel sind dazu jedoch kaum geeignet. Ein weiterer Nachteil der Methylolverbindungen ist die Bildung von Formaldehyd, die zu einer Belastung am Arbeitsplatz führt.

Weitere Verfahren zur Behandlung von Cellulosefasern der Gattung Lyocell sind z.B. aus der WO 97/49856, dem Österreichischen Gebrauchsmuster Nr. 2527 und der WO 99/19555 bekannt.

Die im Stand der Technik zur Behandlung von Cellulosefasern der Gattung Lyocell vorgeschlagenen Verfahren weisen jedoch einige Nachteile auf:

So sind zahlreiche der bisher vorgeschlagenen Textilhilfsmittel nur sehr aufwendig herzustellen und daher teuer. Bei anderen Textilhilfsmitteln ist zu beobachten, daß sie in dem für die Reaktion mit der Cellulose notwendigen alkalischen Milieu hydrolyseanfällig sind. Es kommt daher zu Hydrolyseverlusten. Weiters sind viele der vorgeschlagenen Textilhilfsmittel im notwendigen alkalischem Milieu nicht genügend löslich und müssen in Form einer Dispersion eingesetzt werden. Dies kann zu unregelmäßigen Behandlungseffekten führen. Zu erwähnen ist schließlich, daß der Einsatz einiger bekannter Textilhilfsmittel aufgrund ihrer Toxizität schwierig ist.

Die vorliegende Erfindung stellt sich die Aufgabe, ein Verfahren zur Behandlung von Cellulosefasern der Gattung Lyocell und von Gebilden aus diesen Fasern zur Verfügung zu stellen, das auf einfache Weise durchgeführt werden kann und das ermöglicht, daß die behandelten Fasern eine verringerte Fibrillierneigung bzw. behandelte Fasergebilde verbesserte Scheuer- und Pillingwerte aufweisen.

Das erfindungsgemäße Verfahren zur Behandlung von lösungsmittelgesponnenen Fasern, bei welchem die Fasern mit einem Textilhilfsmittel in Kontakt gebracht werden, ist dadurch gekennzeichnet, daß als Textilhilfsmittel eine Verbindung mit der allgemeinen Formel 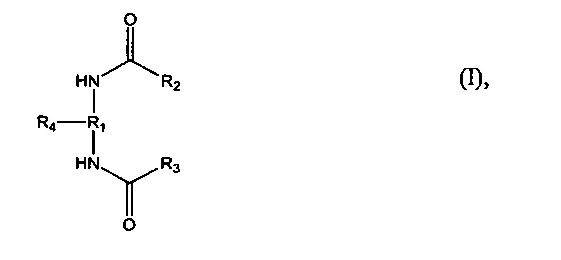 bzw. ein Salz davon eingesetzt wird, wobei
R₁ ein Brückenglied ausgewählt aus der Gruppe bestehend aus einer linearen oder verzweigten Kette mit 1 bis 4 C-Atomen und Phenyl ist,
R₂ und R₃ gleich oder verschieden voneinander sind und aus der Gruppe bestehend aus (CH₂)ₘ-CH=CH₂ mit m = 0 bis 2 ausgewählt sind, und wobei R₄ eine ionische und/oder im alkalischen Milieu ionisierbare Gruppe bedeutet.

Es wurde überraschenderweise gefunden, daß die erfindungsgemäß eingesetzten Textilhilfsmittel, die vergleichsweise kostengünstig erhältlich sind, eine ebenso große bzw. sogar größere Verbesserung der Eigenschaften der behandelten Fasern bewirken wie z.B. die aus der EP-A 0 538 977 bekannten, aufwendig herzustellenden Substanzen.

Weiters wurde gefunden, daß die erfindungsgemäß eingesetzten Substanzen in alkalischem Milieu sehr hydrolysestabil sind. Es kommt daher während der Dauer der Behandlung im Vergleich zu den beispielweise im österreichischen Gebrauchsmuster No. 2527 oder in der WO 99/19555 geoffenbarten Textilhilfsmitteln zu keinen Hydrolyseverlusten. Dadurch wird weniger Textilhilfsmittel benötigt, um gleich gute bzw. sogar bessere Eigenschaften der behandelten Fasern zu erreichen.

In den in der WO 94/09191 bzw. WO 95/28516 beschriebenen Verfahren werden ein 1,3,5-Trisacryloyltetrahydro-1,3,5-triazin sowie N,N'-Methylenbisacrylamid als Textilhilfsmittel eingesetzt. Diese Textilhilfsmittel liegen in nicht-ionischer Form vor. Die erfindungsgemäß eingesetzten Verbindungen liegen demgegenüber in wäßrigem alkalischem Milieu in ionischer und gelöster Form vor.

Die erfindungsgemäß eingesetzten Verbindungen sind auch nur in geringem Ausmaß bzw. überhaupt nicht flüchtig.

Bevorzugt ist der Rest R₄ aus der Gruppe bestehend aus -SO₃H, -COOH, -OH und -SH ausgewählt.

Weiters bevorzugt bedeuten im eingesetzten Textilhilfsmittel R₂ und R₃ beide (CH₂)ₘ-CH=CH₂.

Ein insbesonders bevorzugt eingesetztes Textilhilfsmittel ist eine Verbindung der Formel 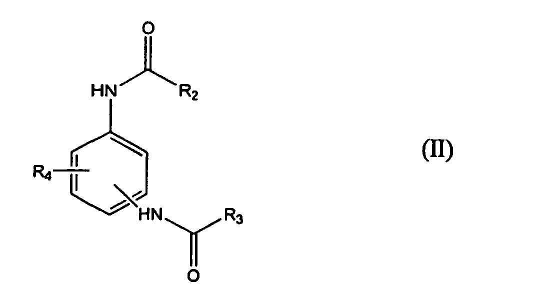 bzw.ein Salz davon.

Weiters bevorzugt wird als Textilhilfsmittel eine Verbindung der Formel 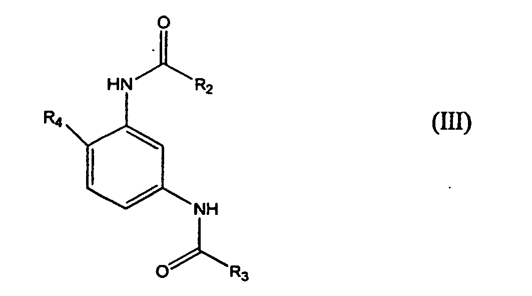 bzw. ein Salz davon eingesetzt.

Insbesondere bevorzugt wird als Textilhilfsmittel eine Verbindung der Formel 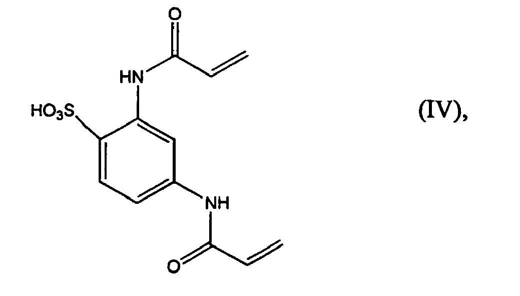 d.h. die 2,4-Bis(acrylamido)benzolsulfonsäure bzw. ein Salz davon eingesetzt.

In einer weiteren bevorzugten Ausführungsform wird als Textilhilfsmittel eine Verbindung der Formel 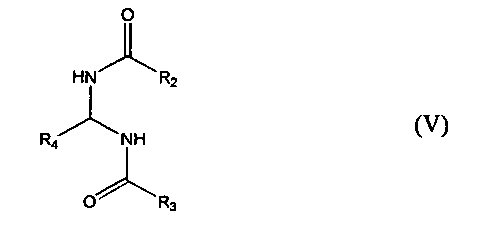 bzw. ein Salz davon eingesetzt.

Insbesondere bevorzugt ist der Einsatz einer Verbindung der Formel 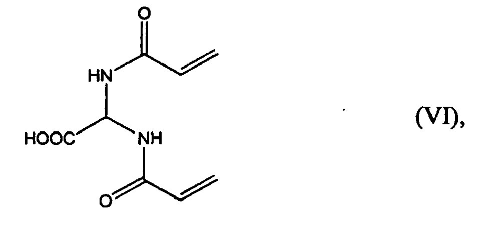 d.h. der Bisacrylamidoessigsäure bzw. eines Salzes davon als Textilhilfsmittel.

Das Textilhilfsmittel wird bevorzugt in Form des Natrium-, Lithium-, Kalium-, Calcium- oder Magnesiumsalzes eingesetzt. Es können aber auch andere Salze, insbesondere Metallsalze eingesetzt werden.

Im erfindungsgemäßen Verfahren werden die Fasern bevorzugt mit einer alkalischen Lösung des Textilhilfsmittels in Kontakt gebracht.

Eine besonders interessante und kostengünstige Variante des erfindungsgemäßen Verfahrens besteht darin, daß eine alkalische Lösung einer Verbindung der Formel 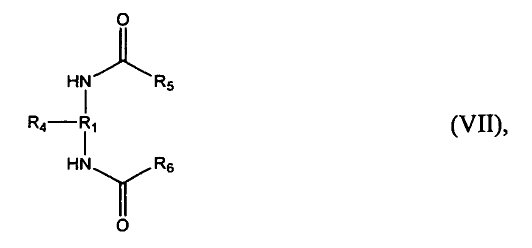 wobei R₅ und R₆ beide (CH₂)ₙ-X, n = 2 bis 4, X=Halogen, hergestellt wird, und diese Verbindung in alkalischer Lösung vor oder während dem In-Kontakt-Bringen mit den Fasern in ein Textilhilfsmittel gemäß Formel (I), in welchem R₂ und R₃ beide (CH₂)ₘ-CH=CH₂, wobei m = n-2, bedeuten, umgesetzt wird.

Es wird somit die erfindungsgemäß eingesetzte Verbindung in der Imprägnierlösung vor oder während dem In-Kontakt-Bringen mit den Fasern gleichsam *in-situ* hergestellt, indem aus der entsprechenden ω-Halogenalkylverbindung die zwei Acrylamidogruppen mittels einer Eliminationsreaktion hergestellt werden.

Im Fall der bevorzugt eingesetzten 2,4-Bis(acrylamido)benzolsulfonsäure (bzw. beispielsweise ihres Natriumsalzes) kann diese in der Imprägnierlösung direkt aus dem Natriumsalz der 2,4-Bis(3-chlorpropionamido)-benzolsulfonsäure hergestellt werden.

Das erfindungsgemäße Verfahren wird bevorzugt zur Behandlung von Fasern durchgeführt, die in niemals getrocknetem Zustand vorliegen.

Zur kontinuierlichen Behandlung von niemals getrockneten lösungsmittelgesponnen Fasern können die geschnittenen, von NMMO frei gewaschenen Fasern mit einer z.B. durch Abpressen eingestellten definierten Feuchte von 50% bis 500% in einem losen Verbund ("Vlies") auf einem bewegten Siebband mit der das Textilhilfsmittel enthaltenden Flotte in Kontakt gebracht und z.B. durch Besprühen durchtränkt werden ("Imprägnierung"). Das Vlies hat ein Flächengewicht von 0,2-15 kg/m², vorzugsweise zwischen 1-8 kg/m² bezogen auf die trockene Faser, wobei die Verweilzeit der Faser in diesem Imprägnierungsfeld zwischen 1-25 min betragen kann, vorzugsweise aber 2 bis 10 min. Die Imprägnierung der Faser kann nur von einer Seite, aber auch von beiden Seiten des Vlieses aus erfolgen, wobei das Verhältnis Faser zu Behandlungsflotte (kg/kg) von 1:1 bis 1:50 betragen kann. Das Textilhilfsmittel kann dabei in Mischung mit oder getrennt vom gegebenenfalls erforderlichen Katalysator aufgebracht werden.

Nach der Imprägnierung wird das Vlies auf eine definierte Feuchte von 50% bis 500% abgepreßt, die abgepreßte Behandlungsflotte in den Imprägnierungskreislauf zurückgeführt und das Vlies mittels geeigneter Einrichtungen, vorzugsweise durch heißen Dampf, auf Temperatur gebracht und gehalten, wobei die Verweilzeit etwa 2-15 min betragen kann. Dabei erhitzt sich die Faser mit der Flotte auf die gewünschte Temperatur, und die Cellulose reagiert mit dem Textilhilfsmittel. Vorzugsweise wird die Behandlung so geführt, daß die Faser dabei nicht trocknet.

Anschließend wird die Faser von nicht fixierten Reaktionsprodukten möglichst unter Neutralisation freigewaschen. Bei alkalisch katalysierten Vernetzungsmitteln kann dazu in den ersten Waschschritten eine sauer gestellte Waschflotte verwendet werden. Die Anzahl der Waschstufen richtet sich nach den eingesetzten Konzentrationen bzw. nach der Wascheffizienz der einzelnen Stufen.

Die erfindungsgemäß eingesetzten Textilhilfsmittel eignen sich in hervorragender Weise auch zur Behandlung von bereits getrockneten Fasern und zur Behandlung von Fasergebilden, wie z.B. Garnen, Geweben oder Gestricken, die lösungsmittelgesponnene Fasern enthalten. Die Fasern können in gefärbter oder ungefärbter Form vorliegen.

Die vorliegende Erfindung betrifft ferner die Verwendung einer Verbindung der Formel 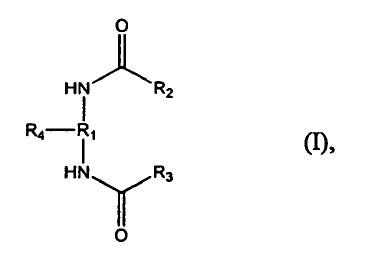 wobei R₁, R₂, R₃ und R₄ die oben angegebene Bedeutung haben, bzw. eines Salzes davon, als Textilhilfsmittel zur Behandlung von lösungsmittelgesponnenen Fasern.

Die Verbindungen der Formel (I) eignen sich dabei insbesondere zur Verringerung der Fibrillationstendenz von lösungsmittelgesponnenen Fasern.

Es wurde jedoch auch überraschenderweise gefunden, daß die Verbindungen der Formel (I), insbesondere jene, in welchen R₁ Phenyl ist, die Erhöhung der UV-Absorption von lösungsmittelgesponnenen Fasern bewirken.

Die vorliegende Erfindung betrifft ferner lösungsmittelgesponnene Fasern, die nach dem erfindungsgemäßen Verfahren erhältlich sind sowie Fasergebilde, welche solche lösungsmittelgesponnenen Fasern enthalten.

Die erfindungsgemäß eingesetzten Textilhilfsmittel, bei denen der Rest R₁ Phenyl ist, sind neue Verbindungen. Die Erfindung betrifft daher weiters eine Verbindung der Formel 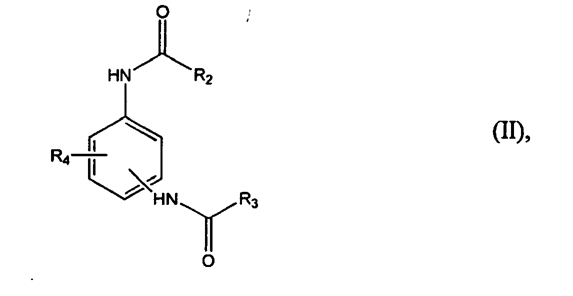 wobei R₂, R₃ und R₄ jeweils die oben angegebene Bedeutung haben, sowie deren Salze, mit den Maßgaben, daß
- wenn R₄ = -OH und R₂ und R₃ beide -CH=CH₂, befinden sich die beiden Amidgruppen nicht in para-Stellung zueinander
- wenn R₄ = -SO₃H, R₂ und R₃ beide -CH=CH₂ und die beiden Amidgruppen befinden sich in ortho-Stellung zueinander, ist das Calciumsalz ausgeschlossen.

Das N,N'-(2-hydroxy-1,4-phenylen)-bis-2-propenamid ist unter der RN-Nummer 105298-66-6 in den "Chemical Abstracts" (American Chemical Society) registriert.

Das Calciumsalz der 3,4-bis-[(1-oxo-2-propenyl)amino]benzolsulfonsäure ist unter der RN-Nummer 58772-33-1 in den "Chemical Abstracts" (American Chemical Society) registriert.

Die erfindungsgemäßen Verbindungen können dadurch hergestellt werden, daß eine Verbindung der Formel 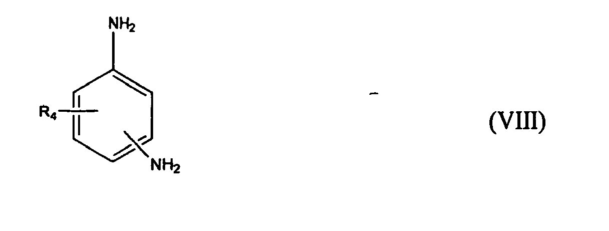 mit einer Verbindung der Formel 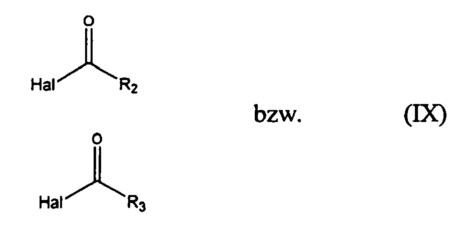 worin R₂, R₃ und R₄ jeweils die oben angegebenen Bedeutungen haben, umgesetzt wird und das erhaltene Produkt gegebenenfalls in Form eines Salzes gebracht wird.

Die Verbindungen der Formel (II) können dadurch hergestellt werden, daß eine Verbindung der Formel 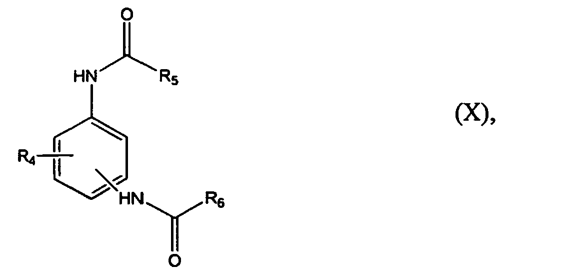 wobei R₅ und R₆ beide (CH₂)ₙ-X, n = 2 bis 4, X = Halogen, in alkalischer Lösung zu einer Verbindung gemäß Formel (II), in welcher R₂ und R₃ beide (CH₂)ₘ-CH=CH₂, wobei m=n-2, bedeuten, umgesetzt wird.

Auch die Verbindungen der Formel (X) sind neu. Sie können dadurch hergestellt werden, daß eine Verbindung der Formel 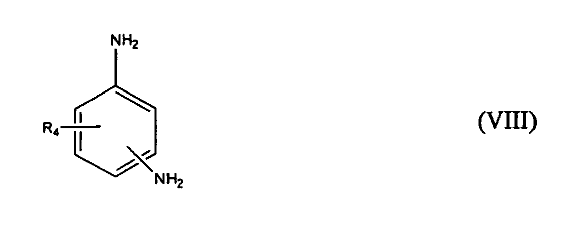 mit einer Verbindung der Formel 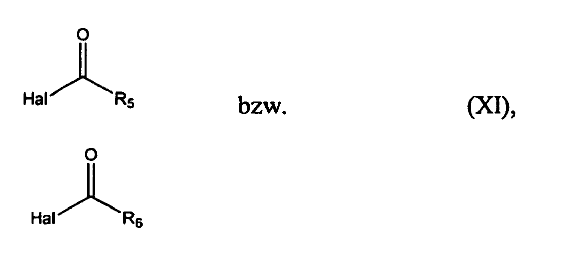 wobei R₄, R₅ und R₆ die oben angegebenen Bedeutungen haben, umgesetzt wird und das erhaltene Produkt gegebenenfalls in Form eines Salzes gebracht wird.

### Beispiele:

### Beispiel 1 - Herstellung von 2,4-Bis(acrylamido)benzolsulfonsäure-Natriumsalz:

In einem 51-Becherglas mit mechanischer Rührung wurden 66g KH₂PO₄ und 3g Na₂HPO₄ in 1,51 H₂O gelöst (pH=6,4). Nach Zugabe von 100g (=0,53 mol) 2,4-Diaminobenzolsulfonsäure wurde die Lösung durch Einbringen von Eis auf <1°C gekühlt. Über einen Tropftrichter wurden innerhalb von 70min 260ml (=233g =2,58 mol) Acrylsäurechlorid zugetropft und durch Zugabe von 20%iger NaOH-Lösung der pH-Wert auf ca. 3,5 eingestellt (pH-Werte unter 3 und über 4 sind zu vermeiden). Die Temperatur der Lösung wurde durch Einbringen von Eis auf <1°C gehalten. Nach beendeter Zugabe (Endvolumen = 51) wurde noch 1h gerührt, wobei man die Lösung auf Raumtemperatur (RT) erwärmen ließ.

Nach Neutralisierung mit NaOH wurde die geringe Menge an weißem Feststoff von der schwarzen Lösung abfiltriert, dem Filtrat soviel NaCl zugegeben, daß eine 20%ige Lösung entsteht und über Nacht langsam gerührt. Der entstandene graue Niederschlag wurde abfiltriert, mit 20%iger NaCl-Lösung gewaschen und im Trockenschrank bei ca. 65°C getrocknet (ca. 24h).

Das Endprodukt wurde mittels HPLC und NMR-Spektroskopie analysiert.

Ausbeute: 152g (=90% d. Th.) 2,4-Bis(acrylamido)benzolsulfonsäure-Natriumsalz.

### Beispiel 2 - Herstellung von 2,4-Bis(3-chlor-propionamido)-benzolsulfonsäure-Natriumsalz:

38,8g 2,4-Diaminobenzolsulfonsäure (Hersteller BASF) wurden in 600ml Wasser dispergiert und gerührt. Der pH-Wert wurde mit gelöster NaOH auf 6,5 gestellt. Nach Kühlung auf 10°C wurden 5,3g 3-Chlor-propionylchlorid tropfenweise zugegeben (0,5h), wobei die Temperatur auf 5-10°C und der pH-Wert auf 3,0-3,5 gehalten wurde. Nach Fertigstellung der Zugabe wurde mittels Dünnschichtchromatographie festgestellt, daß noch Spuren des monoacylierten Produktes vorhanden waren. Es wurden daher weitere 13g Säurechlorid zugegeben, um die Reaktion fertigzustellen. Nach Anheben des pH-Wertes auf 5 wurde mit 20% w/v NaCl-Lösung ausgesalzen und der gebildete graue Feststoff abfiltriert. Der Kuchen wurde mit 150ml Kochsalzlösung (20% w/v) gewaschen und bei 65°C ofengetrocknet, woraus 146g Produkt erhalten wurden.

### Beispiel 3:

Gemäß dem in der WO 93/19230 beschriebenen Verfahren hergestellte lösungsmittelgesponnene Fasern mit einem Titer von 1,3 dtex wurden in niemals getrocknetem Zustand wie folgt behandelt:

Die Fasern wurden bei einem Flottenverhältnis von 1:10 mit einer Lösung, enthaltend 50g/l Natriumsalz der 2,4-Bis(acrylamido)-benzolsulfonsäure und 100 g/l Natriumsulfat 4 Minuten bei 30°C imprägniert. Dann wurden 4g/l NaOH zugesetzt und weitere 2 Minuten imprägniert. Die Lösung hatte einen pH-Wert von 12,7. Anschließend wurden die Fasern mittels eines Quetschwerkes auf einen Feuchtigkeitsgehalt von 100%-110% abgequetscht und 4 Minuten mit Sattdampf (100%) bei 100°C wärmebehandelt, ausgewaschen und getrocknet.

An den Fasern wurde der Fibrillationsgrad anhand der Naßscheuerbeständigkeit (Prüfmethode gemäß WO 99/19555) der Einzelfasern bestimmt. Nach dieser Testmethode ergibt sich für die erfindungsgemäß behandelte Probe im Mittel ein Wert von 560 Umdrehungen. Eine unbehandelte Faser zeigt im Vergleich dazu einen Naßscheuerwert von lediglich 40-60 Umdrehungen. Der Stickstoffgehalt der behandelten Fasern beträgt 0,34%.

### Beispiel 4:

Niemals getrocknete lösungsmittelgesponnene Fasern mit einem Titer von 1,3 dtex wurden bei einem Flottenverhältnis von 1:10 mit einer Lösung, enthaltend 50g/l Natriumsalz der 2,4-Bis(acrylamido)-benzolsulfonsäure und 50g/l Natriumsulfat, der kurz vor der Imprägnierung 4g/l NaOH zugesetzt worden waren, 2 Minuten lang bei Raumtemperatur (RT) imprägniert (pH-Wert der Lösung 12,7). Dann wurden die Fasern mittels eines Quetschwerkes (Foulard) auf einen Feuchtigkeitsgehalt von 100%-110% abgequetscht, 6 Minuten mit Sattdampf wärmebehandelt, ausgewaschen und getrocknet.

Die Ermittlung der Naßscheuerbeständigkeit der so behandelten Fasern ergab im Mittel einen Wert von 1180 Umdrehungen. Der Stickstoffgehalt der Fasern beträgt 0,38%.

### Beispiel 5 (in-situ-Generierung von 2,4-Bis(acrylamido)-benzolsulfonsäure):

Eine Lösung, enthaltend 50g/l Natriumsalz der 2,4-Bis(3-chlorpropionamido)-benzolsulfonsäure wird bei 30°C mit 10g/l Natriumhydroxid versetzt und 20 Minuten bei dieser Temperatur gerührt. In dieser Lösung wurden niemals getrocknete lösungsmittelgesponnene Fasern (Titer 1,3 dtex) bei einem Flottenverhältnis von 1:10 über 2 Minuten bei 30°C imprägniert. Dann wurden 100g/l Natriumsulfat in der Imprägnierlösung gelöst und nach 2 Minuten 4g/l NaOH zugesetzt. Nach weiteren 4 Minuten Imprägnierung bei 30°C wurden die Fasern auf eine Flottenaufnahme von 100%-110% abgequetscht, 4 Minuten mit Sattdampf wärmebehandelt, ausgewaschen und getrocknet.

Die Ermittlung der Naßscheuerbeständigkeit der so behandelten Fasern ergab im Mittel einen Wert von 780 Umdrehungen. Der Stickstoffgehalt der Fasern beträgt 0,34%.

### Beispiel 6 (in-situ-Generierung von 2,4-Bis(acrylamido)-benzolsulfonsäure):

Eine Lösung, enthaltend 50g/l Natriumsalz der 2,4-Bis(3-chlorpropionamido)-benzolsulfonsäure wurde bei 30°C mit 10g/l Natriumhydroxid versetzt und 20 Minuten bei dieser Temperatur gerührt. In dieser Lösung wurden 100g/l Natriumsulfat und 4g/l NaOH gelöst. Ein Stück eines Vlieses aus niemals getrockneten lösungsmittelgesponnenen Fasern (Titer 1,3 dtex) wurde in dieser Lösung 4 Minuten lang bei einem Flottenverhältnis von 1:10 bei 30°C imprägniert. Dann wurde das Vliesstück auf eine Flottenaufnahme von 100%-110% abgequetscht, 4 Minuten mit Sattdampf wärmebehandelt, ausgewaschen und getrocknet.

Die Ermittlung der Naßscheuerbeständigkeit der so behandelten Fasern ergab im Mittel einen Wert von 570 Umdrehungen. Der Stickstoffgehalt der Fasern beträgt 0,39%.

### Beispiel 7:

Ein gefärbtes Gestrick aus lösungsmittelgesponnenen Fasern wurde in einem Flottenverhältnis von 1:30 mit einer wäßrigen Lösung, enthaltend 50g/l Natriumsalz der 2,4-Bis(acrylamido)-benzolsulfonsäure und 50g/l Natriumsulfat, der kurz vor der Imprägnierung 4g/l NaOH zugesetzt worden waren, 2 Minuten lang bei Raumtemperatur (RT) imprägniert (pH-Wert der Lösung 12,7). Die überschüssige Lösung wurde mit einem Foulard bei 1 bar abgepreßt. Das Gestrick wurde 5 Minuten bei 100°C mit Wasserdampf wärembehandelt. Anschließend wurde das Gestrick mit 2%iger Essigsäure und Wasser wiederholt gewaschen und abschließend getrocknet.

Aus dem Gestrick wurden einzelne Fasern präpariert und einer Naßscheuerprüfung unterzogen. Der Mittelwert der Naßscheuerprüfung betrug 650 Umdrehungen.

### Beispiel 8:

Eine ungefärbtes Gestrick aus lösungsmittelgesponnenen Fasern wurde wie in Beispiel 7 beschrieben behandelt und einer Naßscheuerprüfung unterzogen. Der Mittelwert der Naßscheuerprüfung betrug 620 Umdrehungen.

### Beispiel 9:

Eine Lösung enthaltend 60 g/l Bisacrylamidoessigsäure wurde bei 30°C mit 21 g/l Natriumhydroxid versetzt, gerührt, und in diese Lösung wurden sofort niemals getrocknete lösungsmittelgesponnene Fasern mit einem Titer von 1,3dtex bei einem Flottenverhältnis von 1:10 zugegeben und dann 2 min. bei 50°C imprägniert. Dann wurde in diese Lösung 100 g/l Natriumsulfat zugegeben und weitere 3 min. bei 50°C imprägniert. Dann wurde auf eine Flottenaufnahme von 100-110% abgequetscht und 4 Minuten mit Sattdampf wärmebehandelt, ausgewaschen und getrocknet.

Die Naßscheuerbeständigkeit an der Einzelfaser ergab im Mittel einen Wert von 700 Umdrehungen. Der Stickstoffgehalt der Faser betrug 0,33%.

### Beispiel 10:

Eine Lösung enthaltend 60 g/l Bisacrylamidoessigsäure wurde bei Raumtemperatur mit 21 g/l Natriumhydroxid versetzt, gerührt, und in diese Lösung wurden sofort niemals getrocknete lösungsmittelgesponnene Fasern mit einem Titer von 1,3dtex bei einem Flottenverhältnis von 1:10 zugegeben und dann 2 min. bei Raumtemperatur imprägniert. Dann wurden in diese Lösung 100 g/l Natriumsulfat zugegeben und weitere 3 min. bei 50°C imprägniert. Dann wurde auf eine Flottenaufnahme von 100-110% abgequetscht und 4 Minuten mit Sattdampf wärmebehandelt, ausgewaschen und getrocknet.

Die Naßscheuerbeständigkeit an der Einzelfaser ergab im Mittel einen Wert von 400 Umdrehungen. Der Stickstoffgehalt betrug 0,32%.

### Beispiel 11:

Eine Lösung enthaltend 60 g/l Bisacrylamidoessigsäure wurde bei Raumtemperatur mit 21 g/l Natriumhydroxid versetzt, gerührt, und in diese Lösung wurden sofort niemals getrocknete lösungsmittelgesponnene Fasern mit einem Titer von 1,3dtex bei einem Flottenverhältnis von 1:10 zugegeben und dann 5 min. bei Raumtemperatur imprägniert. Dann wurde auf eine Flottenaufnahme von 100-110% abgequetscht und 4 Minuten mit Sattdampf wärmebehandelt, ausgewaschen und getrocknet.

Die Naßscheuerbeständigkeit an der Einzelfaser ergab im Mittel einen Wert von 320 Umdrehungen. Der Stickstoffgehalt der Faser betrug 0,31 %.

## Patentansprüche

1. Verfahren zur Behandlung von lösungsmittelgesponnenen cellulosischen Fasern, bei welchem die Fasern mit einem Textilhilfsmittel in alkalischem Milieu in Kontakt gebracht werden, **dadurch gekennzeichnet, daß** als Textilhilfsmittel eine Verbindung mit der allgemeinen Formel 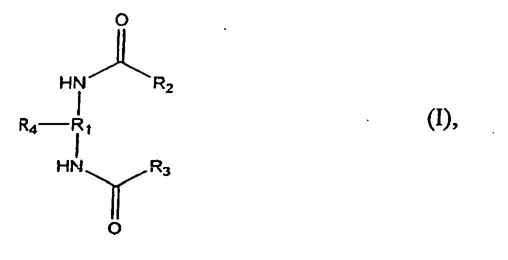 bzw. ein Salz davon eingesetzt wird, wobei
R₁ ein Brückenglied ausgewählt aus der Gruppe bestehend aus einer linearen oder verzweigten Kette mit 1 bis 4 C-Atomen und Phenyl ist,
R₂ und R₃ gleich oder verschieden voneinander sind und aus der Gruppe bestehend aus (CH₂)ₘ-CH=CH₂ mit m = 0 bis 2 ausgewählt sind, und wobei R₄ eine ionische und/oder in alkalischem Milieu ionisierbare Gruppe bedeutet.

2. Verfahren gemäß Anspruch 1, **dadurch gekennzeichnet, daß** R₄ aus der Gruppe bestehend aus -SO₃H, -COOH, -OH und -SH ausgewählt ist.

3. Verfahren gemäß Anspruch 1 oder 2, **dadurch gekennzeichnet, daß** im eingesetzten Textilhilfsmittel R₂ und R₃ beide (CH₂)ₘ -CH=CH₂ bedeuten.

4. Verfahren gemäß einem der Ansprüche 1 bis 3, **dadurch gekennzeichnet, daß** als Textilhilfsmittel eine Verbindung der Formel 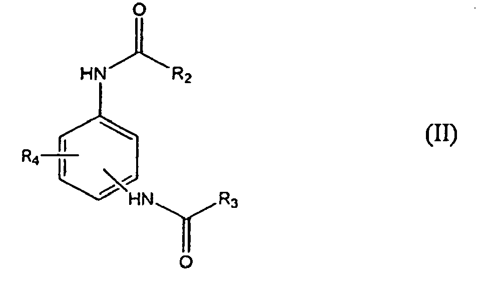 bzw ein Salz davon eingesetzt wird.

5. Verfahren gemäß Anspruch 4, **dadurch gekennzeichnet, daß** als Textilhilfsmittel eine Verbindung der Formel 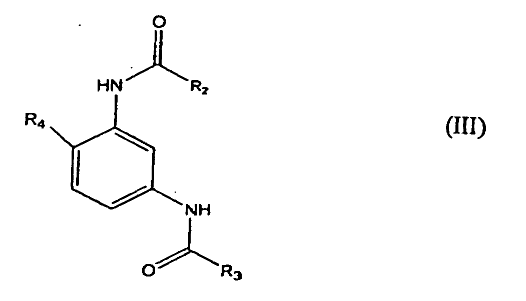 bzw. ein Salz davon eingesetzt wird.

6. Verfahren gemäß Anspruch 5, **dadurch gekennzeichnet, daß** als Textilhilfsmittel eine Verbindung der Formel 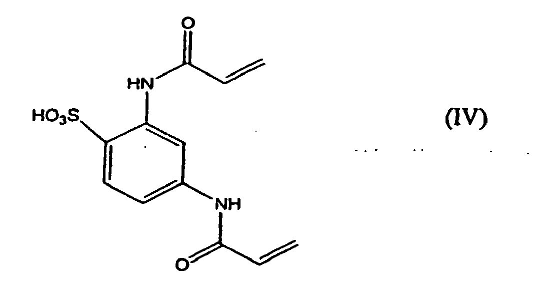 bzw. ein Salz davon eingesetzt wird.

7. Verfahren gemäß einem der Ansprüche 1 bis 3, **dadurch gekennzeichnet, daß** als Textilhilfsmittel eine Verbindung der Formel 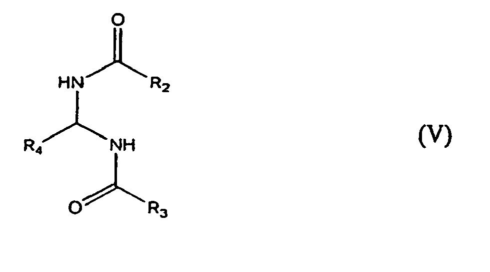 bzw. ein Salz davon eingesetzt wird.

8. Verfahren gemäß Anspruch 7, **dadurch gekennzeichnet, daß** als Textilhilfsmittel eine Verbindung der Formel 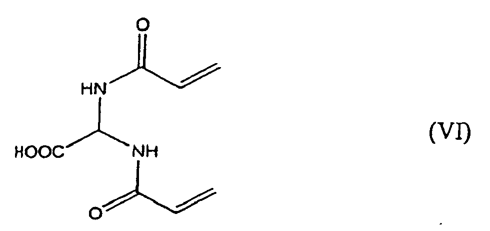 bzw. ein Salz davon eingesetzt wird.

9. Verfahren gemäß einem der Anspüche 1 bis 8, **dadurch gekennzeichnet, daß** das Textilhilfsmittel in Form des Natrium-, Lithium-, Kalium-, Calcium- oder Magnesiumsalzes eingesetzt wird.

10. Verfahren gemäß einem der Ansprüche 1 bis 9, **dadurch gekennzeichnet, daß** die Fasern mit einer alkalischen Lösung des Textilhilfsmittels in Kontakt gebracht werden.

11. Verfahren gemäß einem der Ansprüche 1 bis 6, **dadurch gekennzeichnet, daß** eine alkalische Lösung einer Verbindung der Formel 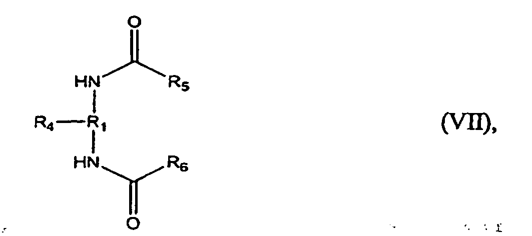 wobei R₅ und R₆ beide (CH₂)ₙ-X, n = 2 bis 4, X=Halogen, hergestellt wird, und diese Verbindung in alkalischer Lösung vor oder während dem In-Kontakt-Bringen mit den Fasern in ein Textilhilfsmittel gemäß Formel (I), in welchem R₂ und R₃ beide (CH₂)ₘ-CH=CH₂, wobei m = n-2, bedeuten, umgesetzt wird.

12. Verfahren gemäß einem der Ansprüche 1 bis 11, **dadurch gekennzeichnet, daß** die Fasern in niemals getrocknetem Zustand vorliegen.

13. Verfahren gemäß einem der Ansprüche 1 bis 11, **dadurch gekennzeichnet, daß** die Fasern als Fasergebilde bzw. als Bestandteil eines Fasergebildes vorliegen.

14. Verwendung einer Verbindung der Formel 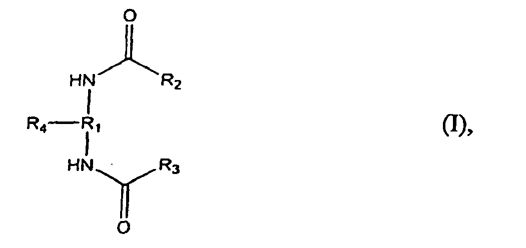 wobei R₁, R₂, R₃ und R₄ die in Anspruch 1 angegebene Bedeutung haben, bzw. eines Salzes davon, als Textilhilfsmittel zur Behandlung von lösungsmittelgesponnenen Cellulosefasern.

15. Verwendung gemäß Anspruch 14 zur Verringerung der Fibrillationstendenz von lösungsmittelgesponnenen Cellulosefasern.

16. Verwendung gemäß Anspruch 14 oder 15 zur Erhöhung der UV-Absorption von lösungsmittelgesponnenen Cellulosefasern.

17. Lösungsmittelgesponnene Cellulosefasern, erhältlich nach einem Verfahren gemäß einem der Ansprüche 1 bis 13.

18. Fasergebilde enthaltend lösungsmittelgesponnene Cellulosefasern, erhältlich nach einem Verfahren gemäß Anspruch 13.

## Claims

1. A process for treating solvent-spun, cellulosic fibres, wherein the fibres are contacted with a textile auxiliary agent in an alkaline environment, **characterized in that** a compound of the general formula 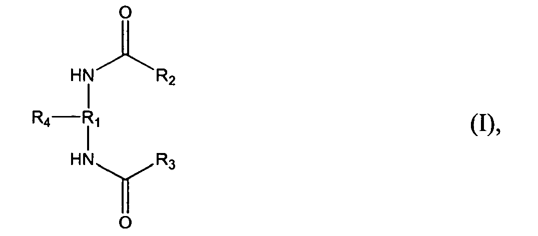 or a salt thereof, respectively, is used as the textile auxiliary agent, wherein
R₁ is a bridging link selected from the group consisting of a linear or branched chain comprising 1 to 4 C-atoms and phenyl,
R₂ and R₃ are the same or different and are selected from the group consisting of (CH2)m-CH=CH₂ with m = 0 to 2, and wherein R₄ denotes a ionic group and/or a group ionizable in an alkaline environment.

2. A process according to claim 1, **characterized in that** R₄ is selected from the group consisting of -SO₃H, -COOH, -OH and -SH.

3. A process according to claim 1 or 2, **characterized in that**, in the textile auxiliary agent used, R₂ and R₃ both mean (CH₂)ₘ-CH=CH₂.

4. A process according to any one of claims 1 to 3, **characterized in that** a compound of the formula 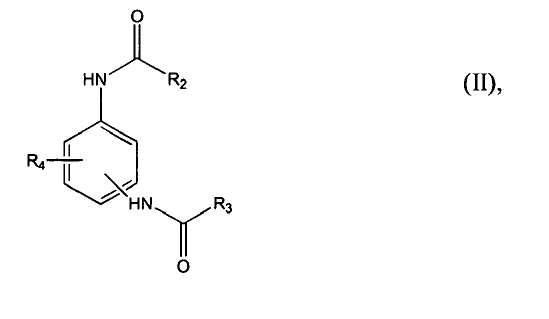 or a salt thereof, respectively, is used as a textile auxiliary agent.

5. A process according to claim 4, **characterized in that** a compound of the formula 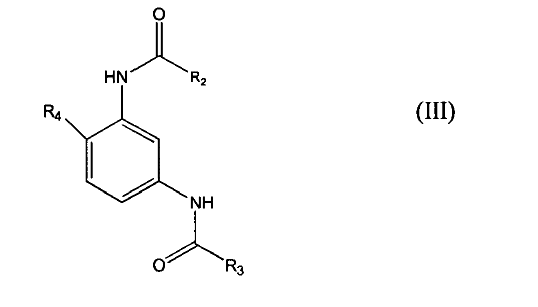 or a salt thereof, respectively, is used as a textile auxiliary agent.

6. A process according to claim 5, **characterized in that** a compound of the formula 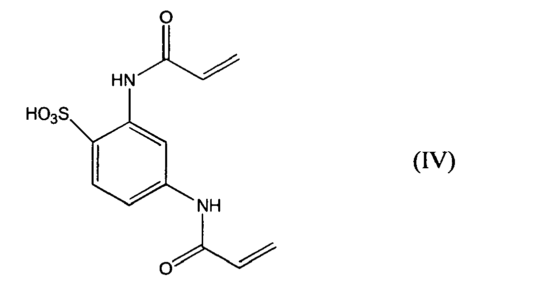 or a salt thereof, respectively, is used as a textile auxiliary agent.

7. A process according to any one of claims 1 to 3, **characterized in that** a compound of the formula 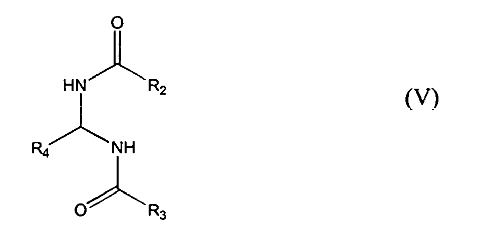 or a salt thereof, respectively, is used as a textile auxiliary agent.

8. A process according to claim 7, **characterized in that** a compound of the formula 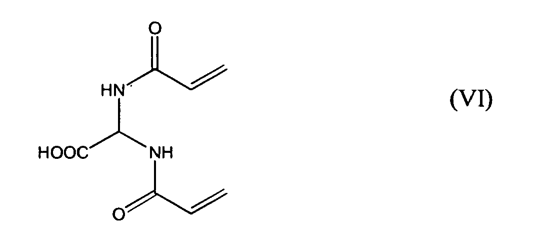 or a salt thereof, respectively, is used as a textile auxiliary agent.

9. A process according to any one of claims 1 to 8, **characterized in that** the textile auxiliary agent is used in the form of the sodium, lithium, potassium, calcium or magnesium salt.

10. A process according to any one of claims 1 to 9, **characterized in that** the fibres are contacted with an alkaline solution of the textile auxiliary agent.

11. A process according to any one of claims 1 to 6, **characterized in that** an alkaline solution of a compound of the formula 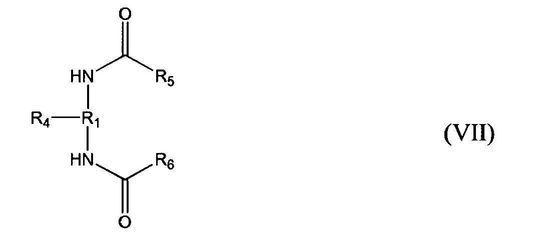 wherein R₅ and R₆ both are (CH₂)ₙ-X, n=2 to 4, X=halogen, is produced and that, prior to or during contacting with the fibres, this compound in an alkaline solution is reacted to a textile auxiliary agent according to formula (I), wherein R₂ and R₃ both mean (CH₂)ₘ-CH=CH₂, wherein m = n-2.

12. A process according to any one of claims 1 to 11, **characterized in that** the fibres are provided in the never-dried state.

13. A process according to any one of claims 1 to 11, **characterized in that** the fibres are provided as fibre assemblies or as a component of a fibre assembly, respectively.

14. The use of a compound of the formula 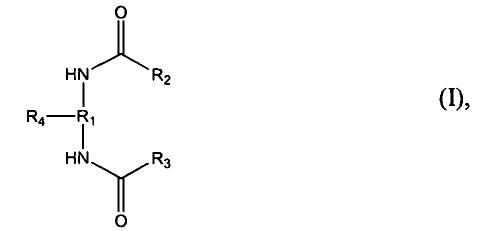 wherein R₁, R₂, R₃ and R₄ have the meaning indicated in claim 1, or of a salt thereof, respectively, as a textile auxiliary agent for the treatment of solvent-spun cellulose fibres.

15. The use according to claim 14 for decreasing the tendency to fibrillation of solvent-spun cellulose fibres.

16. The use according to claim 14 or 15 for increasing the ultraviolet absorption of solvent-spun cellulose fibres.

17. Solvent-spun cellulose fibres, obtainable by a process according to any one of claims 1 to 13.

18. A fibre assembly containing solvent-spun cellulose fibres, obtainable in accordance with a process according to claim 13.

## Revendications

1. Procédé pour traiter des fibres cellulosiques filées à l'aide d'un solvant, dans lequel les fibres sont amenées en contact avec un adjuvant textile dans un milieu alcalin, **caractérisé en ce qu'**un composé de formule générale 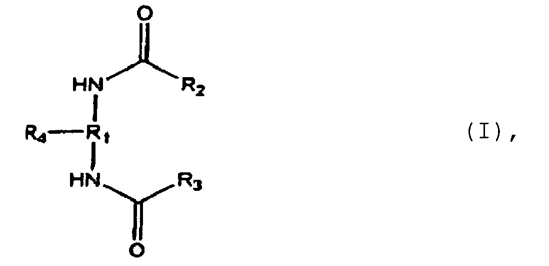 ou un sel de celui-ci est mis en oeuvre en tant qu'adjuvant textile, formule dans laquelle
R₁ représente un élément de pontage choisi dans le groupe se composant d'une chaîne linéaire ou ramifiée ayant de 1 à 4 atomes de C et d'un groupe phényle,
R₂ et R₃ sont identiques ou différents l'un de l'autre et sont choisis dans le groupe se composant du groupe (CH₂)ₘ-CH=CH₂ avec m = 0 à 2, et dans laquelle R₄ signifie un groupe ionique et/ou ionisable dans un milieu alcalin.

2. Procédé selon la revendication 1, **caractérisé en ce que** R₄ est choisi dans le groupe se composant des groupes -SO₃H, -COOH, -OH et -SH.

3. Procédé selon la revendication 1 ou 2,
**caractérisé en ce que** dans l'agent textile mis en oeuvre, R₂ et R₃ représentent tous deux un groupe (CH₂)ₘ-CH=CH₂.

4. Procédé selon l'une quelconque des revendications 1 à 3, **caractérisé en ce qu'**un composé de formule 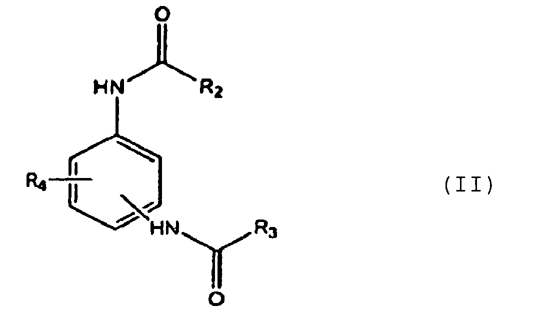 ou un sel de celui-ci est mis en oeuvre en tant qu'adjuvant textile.

5. Procédé selon la revendication 4, **caractérisé en ce qu'**un composé de formule 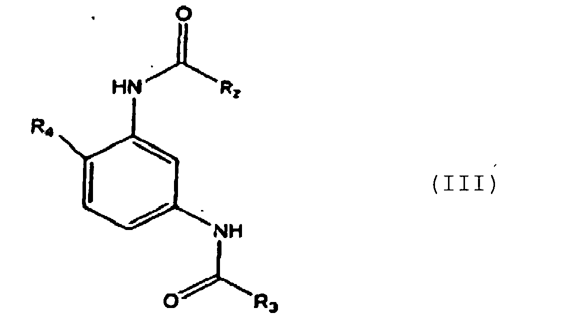 ou un sel de celui-ci est mis en oeuvre en tant qu'adjuvant textile.

6. Procédé selon la revendication 5, **caractérisé en ce qu'**un composé de formule 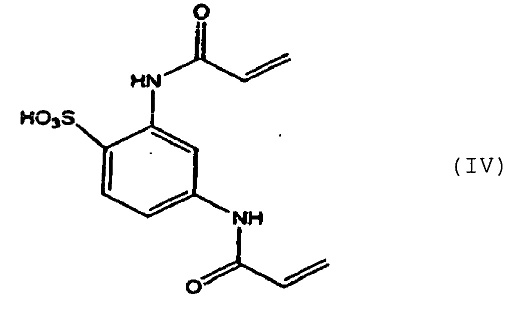 ou un sel de celui-ci est mis en oeuvre en tant qu'adjuvant textile.

7. Procédé selon l'une quelconque des revendications 1 à 3, **caractérisé en ce qu'**un composé de formule 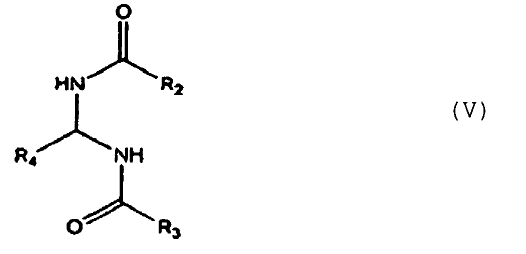 ou un sel de celui-ci est mis en oeuvre en tant qu'adjuvant textile.

8. Procédé selon la revendication 7, **caractérisé en ce qu'**un composé de formule 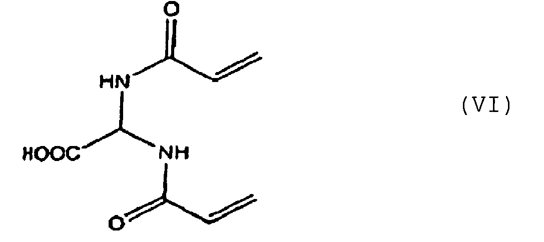 ou un sel de celui-ci est mis en oeuvre en tant qu'adjuvant textile.

9. Procédé selon l'une quelconque des revendications 1 à 8, **caractérisé en ce que** l'adjuvant textile est mis en oeuvre sous forme du sel de sodium, lithium, potassium, calcium ou magnésium.

10. Procédé selon l'une quelconque des revendications 1 à 9, **caractérisé en ce que** les fibres sont amenées en contact avec une solution alcaline de l'adjuvant textile.

11. Procédé selon l'une quelconque des revendications 1 à 6, **caractérisé en ce qu'**une solution alcaline d'un composé de formule 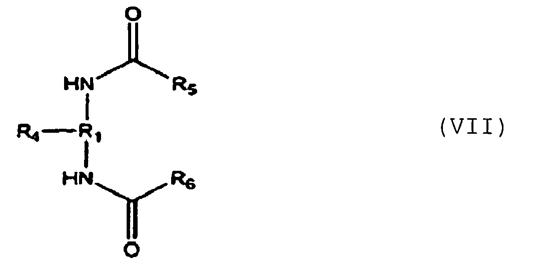 est préparée, formule dans laquelle R₅ et R₆ représentent tous deux un groupe (CH₂)ₘ-X, n= 2 à 4, X représente un atome d'halogène, et ce composé est converti dans une solution alcaline avant ou pendant la mise en contact avec les fibres en un adjuvant textile selon la formule (I), dans laquelle R₂ et R₃ représentent tous deux un groupe (CH₂)ₘ-CH=CH₂, où m = n-2.

12. Procédé selon l'une quelconque des revendications 1 à 11, **caractérisé en ce que** les fibres existent dans un état jamais séché.

13. Procédé selon l'une quelconque des revendications 1 à 11, **caractérisé en ce que** les fibres existent sous forme de structure fibreuse ou sous forme de composant d'une structure fibreuse.

14. Utilisation d'un composé de formule 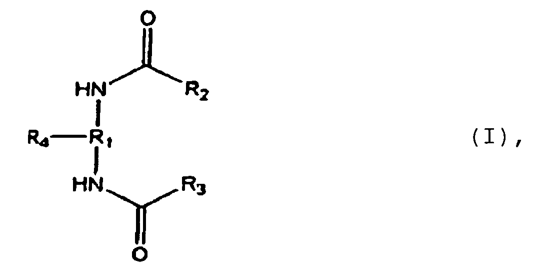 dans laquelle, R₁, R₂, R₃ et R₄ prennent la signification indiquée à la revendication 1, ou d'un sel de celui-ci, en tant qu'adjuvant textile pour le traitement de fibres cellulosiques filées à l'aide d'un solvant.

15. Utilisation selon la revendication 14, pour réduire la tendance à la fibrillation de fibres cellulosiques filées à l'aide d'un solvant.

16. Utilisation selon la revendication 14 ou 15, pour augmenter l'absorption des UV de fibres cellulosiques filées à l'aide d'un solvant.

17. Fibres cellulosiques filées à l'aide d'un solvant, pouvant être obtenues d'après un procédé selon l'une quelconque des revendications 1 à 13.

18. Structure fibreuse contenant des fibres cellulosiques filées à l'aide d'un solvant, pouvant être obtenues d'après un procédé selon la revendication 13.
